# EUROPEAN PATENT APPLICATION

(11) **EP 3 696 041 A1**
(43) Date of publication of application: **19.08.2020**
(21) Application number: 18867196.0
(22) Date of filing: 01.10.2018
(51) Int. Cl.: B60W 40/08, A61B 5/11, B62J 27/00, B62J 99/00

(54) **VEHICLE, DETERMINATION METHOD, AND DETERMINATION PROGRAM**

(30) Priority: 12.10.2017 JP 2017198634
(71) Applicant: Kyocera Corporation, Kyoto-shi Kyoto 612-8501 (JP)
(72) Inventor: TONG, Fangwei, Kyoto-shi Kyoto 612-8501 (JP)
(74) Representative: Viering, Jentschura & Partner mbB Patent- und Rechtsanwälte
(86) International application number: PCT/JP2018/036719
(87) International publication number: WO 2019/073844

(57) **Abstract**

A vehicle includes an information acquisition unit configured to acquire a movement of a body of a driver; and a controller configured to determine whether the movement of the body of the driver acquired by the information acquisition unit is a normal pattern of the driver.

## Description

### CROSS REFERENCE TO RELATED APPLICATION

This application claims priority to and the benefit of Japanese Patent Application No. 2017-198634 filed on October 12, 2017, the entire disclosure of which is incorporated herein by reference.

### TECHNICAL FIELD

The present disclosure relates to a vehicle, a determination method, and a determination program.

### BACKGROUND

Methods of detecting falling of drivers of two-wheeled vehicles are conventionally known. For example, PTL 1 discloses a method of detecting falling of a two-wheeled vehicle based on an image acquired by a camera.

### CITATION LIST

### Patent Literature

PTL 1: JP 2015-107798 A

### SUMMARY

A vehicle according to an aspect comprises an information acquisition unit and a controller. The information acquisition unit is configured to acquire a movement of a body of a driver. The controller is configured to determine whether the movement of the body of the driver acquired by the information acquisition unit is a normal pattern of the driver.

A determination method according to an aspect is performed by a controller, and comprises: acquiring a movement of a body of a driver by an information acquisition unit; and determining whether the movement of the body of the driver acquired by the information acquisition unit is a normal pattern of the driver.

A determination program according to an aspect is configured to cause a computer to: acquire a movement of a body of a driver; and determine whether the movement of the body of the driver acquired is a normal pattern of the driver.

### BRIEF DESCRIPTION OF THE DRAWINGS

In the accompanying drawings:
FIG. 1 is a functional block diagram illustrating an example of a schematic structure of an information processing system according to an embodiment;
FIG. 2 is a schematic side diagram illustrating an example of a vehicle in FIG. 1;
FIG. 3A is a diagram illustrating an example of a pattern of the behavior of the driver (in the case where the driver approaches the seat straight on);
FIG. 3B is a diagram illustrating an example of a pattern of the behavior of the driver (in the case where the driver stops twice for a short time while approaching the seat);
FIG. 4A is a diagram illustrating an example of a pattern of the behavior of the driver (in the case where the driver stops for a long time while approaching the seat);
FIG. 4B is a diagram illustrating an example of a pattern of the behavior of the driver (in the case where the driver repeatedly approaches or moves away from the seat);
FIG. 5 is a diagram illustrating an example of a pattern of the movement of the body surface of the driver;
FIG. 6A is a diagram illustrating an example of a pattern of the movement of the body surface of the driver;
FIG. 6B is a diagram illustrating an example of a pattern of the movement of the body surface of the driver;
FIG. 6C is a diagram illustrating an example of a pattern of the movement of the body surface of the driver;
FIG. 7 is a flowchart illustrating an example of a process performed by a controller in the vehicle in FIG. 1;
FIG. 8 is a flowchart illustrating an example of a process performed by the controller in the vehicle in FIG. 1;
FIG. 9 is a flowchart illustrating a modification of a process performed by the controller in the vehicle in FIG. 1; and
FIG. 10 is a flowchart illustrating an example of a process performed by the controller in the vehicle in FIG. 1.

### DETAILED DESCRIPTION

An embodiment will be described in detail below, with reference to the drawings.

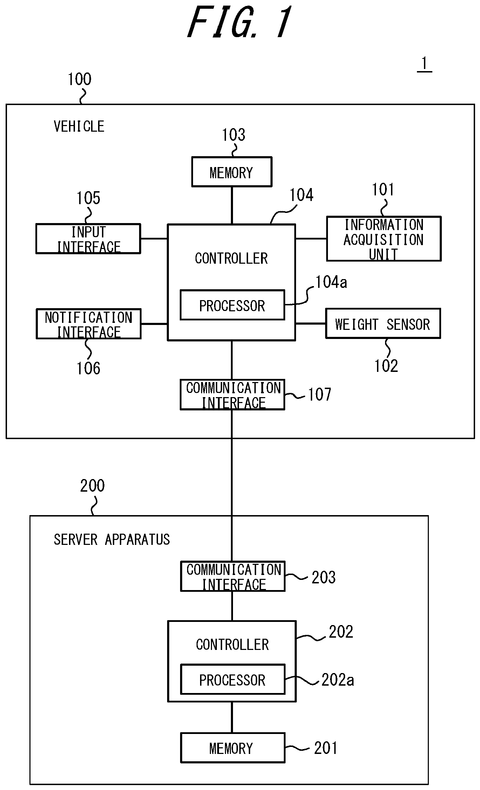

FIG. 1 is a functional block diagram illustrating an example of a schematic structure of an information processing system 1 according to the embodiment. As illustrated in FIG. 1, the information processing system 1 includes a vehicle 100 and a server apparatus 200. The vehicle 100 and the server apparatus 200 are communicably connected to each other.

Examples of the vehicle 100 include automobiles such as electric automobiles, hybrid electric automobiles, and gasoline automobiles, motorcycles such as motorbikes, and bicycles. It is assumed in this embodiment that the vehicle 100 is a motorcycle.

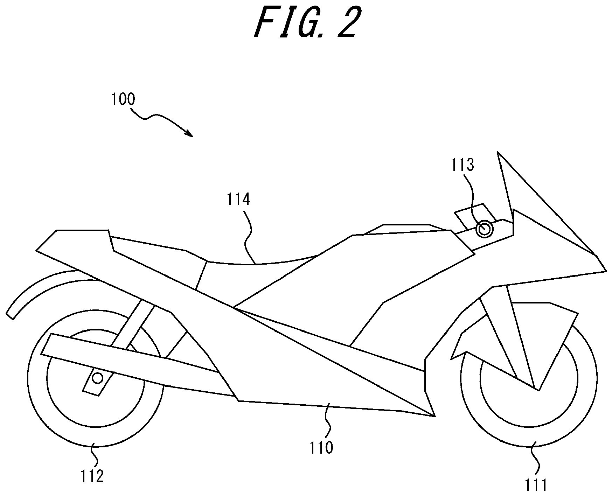

FIG. 2 is a schematic side diagram illustrating an example of the vehicle 100 according to this embodiment. The vehicle 100 includes a vehicle body 110, a front wheel 111 and a rear wheel 112 supporting the vehicle body 110, and a handlebar 113 for steering.

In the vehicle body 110, a fuel tank for storing fuel, an engine for powering the vehicle 100, and the like are arranged. The vehicle body 110 includes a brake pedal that performs operation for braking and a shift pedal that performs operation for shifting gears. The vehicle body 110 also includes a seat 114 on which a driver sits during driving. That is, the driver sits on the seat 114 and drives the vehicle 100. The vehicle body 110 may include a mechanism that implements each functional block in FIG. 1.

The handlebar 113 includes a brake lever that performs operation for braking and a clutch lever that performs clutch operation. For example, the handlebar 113 may be located on the front side of the vehicle body 110.

Referring back to FIG. 1, the vehicle 100 includes an information acquisition unit 101, a weight sensor 102, a memory 103, a controller 104, an input interface 105, a notification interface 106, and a communication interface 107, as functional blocks.

The information acquisition unit 101 acquires the movement of the body of the driver. The information acquisition unit 101 may be configured to detect the body of the driver in all directions in the surroundings of the vehicle 100, or configured to detect the body of the driver only in a specific direction. For example, the information acquisition unit 101 may include a microwave radar. The microwave radar emits microwaves to the surroundings, detects reflected waves of the microwaves, and measures the distance to an object based on the time from the emission of the microwaves to the detection. The microwave radar also measures the direction of the object based on the direction in which the microwaves are detected. The information acquisition unit 101 may include a plurality of microwave radars.

The microwave radar can detect even a slight movement of the body surface, by the Doppler effect. For example, as a result of the microwave radar detecting body motion due to heartbeat, breathing, or the like, the heart rate, the breathing rate, or the like can be determined from the body motion. The information acquisition unit 101 may emit electromagnetic waves to the surroundings, detect reflected waves of the electromagnetic waves, and measure the distance to an object based on the time from the emission of the electromagnetic waves to the detection.

The movement of the body of the driver may include the behavior of the driver. The behavior of the driver includes, for example, the displacement in the direction and distance of the driver relative to the vehicle 100 over time. Specifically, the behavior of the driver may include, for example, the walking speed at which the driver approaches the vehicle 100, the path through which the driver approaches the vehicle 100, and/or the direction from which the driver approaches the vehicle 100. In other words, the movement of the body of the driver includes the change in the position of the driver around the vehicle 100.

The movement of the body of the driver may include the movement of the body surface caused by biological processes of the driver. The biological processes are the driver's organic activity, and may include, for example, heartbeat, breathing, and body motion. The movement of the body surface caused by the biological processes of the driver may include, for example, the movement of the chest due to heartbeat, breathing, body motion, etc.

The information acquisition unit 101 may be located at any position where the movement of the body of the driver can be acquired, in the vehicle body 110. For example, the information acquisition unit 101 may be located near the gauges on the front side of the vehicle body 110.

The information acquisition unit 101 may be a device other than a microwave radar. For example, the information acquisition unit 101 may include a laser range finder, an ultrasonic transceiver, or a camera. The laser range finder can measure a distance based on the time from when laser light is emitted to when reflected light is received. The ultrasonic transceiver can measure a distance based on the time from when ultrasonic waves are emitted to when reflected waves are received. The camera can measure a distance by performing image analysis based on a captured image. The information acquisition unit 101 may include any other device capable of distance measurement. The information acquisition unit 101 may be a combination of a plurality of types of devices.

The information acquisition unit 101 transmits information about the acquired movement of the body of the driver, to the controller 104.

The weight sensor 102 detects a load on the seat 114. For example, the weight sensor 102 is located on the lower side of the seat 114. The weight sensor 102 transmits information about the detected load to the controller 104. The controller 104 can determine whether the driver sits on the seat 114, based on the information about the load acquired from the weight sensor 102.

The memory 103 may be semiconductor memory, magnetic memory, or the like. The memory 103 stores various information, programs for operating the vehicle 100, and the like. The memory 103 may function as working memory. The memory 103 may store the information about the movement of the body of the driver acquired by the information acquisition unit 101, in association with the time of acquisition. The memory 103 may store the load acquired by the weight sensor 102, in association with the time of acquisition.

The memory 103 may store one or more patterns of the movement of the body of the driver. The patterns of the movement of the body of the driver will be described in detail later. The memory 103 may store the result of determination by the controller 104.

The controller 104 includes at least one processor 104a that controls and manages the whole vehicle 100, e.g. each functional block in the vehicle 100. The controller 104 includes at least one processor 104a such as a CPU (Central Processing Unit) that executes a program defining a control procedure, to achieve its functions. Such a program is, for example, stored in the memory 103 or an external storage medium connected to the vehicle 100.

In various embodiments, at least one processor 104a may be implemented as a single integrated circuit (IC), or as a plurality of ICs and/or discrete circuits communicably connected to one another. At least one processor 104a can be implemented according to various known technologies.

In one embodiment, the processor 104a includes, for example, one or more circuits or units configured to perform one or more data calculation procedures or processes by executing instructions stored in related memory. In another embodiment, the processor 104a may be firmware (e.g. a discrete logic component) configured to perform one or more data calculation procedures or processes.

In various embodiments, the processor 104a may include one or more processors, controllers, microprocessors, microcontrollers, application specific integrated circuits (ASICs), digital signal processors, programmable logic devices, field programmable gate arrays, or any combination of these devices or structures, or any combination of other known devices or structures, to perform the functions of the controller 104 described below.

The controller 104 determines whether the movement of the body of the driver acquired by the information acquisition unit 101 is a normal pattern of the driver. For example, the controller 104 determines whether the movement of the body of the driver is a normal pattern of the driver, with reference to each pattern of the movement of the body of the driver stored in the memory 103. The determination process performed by the controller 104 will be described in detail later.

The input interface 105 receives, for example, operation input from the driver. For example, the input interface 105 includes operation buttons (operation keys). The input interface 105 may include a touch screen, with an input region for receiving operation input from the user being displayed in a part of the display device to receive touch operation input by the user. For example, the input interface 105 may be located near the gauges on the front side of the vehicle body 110.

The notification interface 106 notifies information by sound, vibration, images, etc. The notification interface 106 may include a speaker, a vibrator, etc. For example, the notification interface 106 notifies the result of the determination process by the controller 104, based on the control of the controller 104. That is, the notification interface 106 notifies the determination result of whether the movement of the body of the driver is a normal pattern.

The communication interface 107 transmits and receives various information through communication with the server apparatus 200. The communication interface 107 can perform information transmission and reception using a wireless network, a wire network, or a combination thereof. The communication interface 107 can perform communication using, for example, Bluetooth® (Bluetooth is a registered trademark in Japan, other countries, or both), infrared, near field radio communication (NFC), wireless local area network (LAN), wire LAN, or any other communication medium, or any combination thereof.

The server apparatus 200 includes, for example, a computer. The server apparatus 200 acquires information from the vehicle 100, and stores the acquired information. The server apparatus 200 may provide (transmit) the stored information to, for example, a terminal apparatus (not illustrated). Examples of the terminal apparatus include a mobile phone, a smartphone, and a tablet terminal. The terminal apparatus may be, for example, a terminal apparatus possessed by a relevant party having a predetermined relationship with the driver. Examples of the relevant party include the driver's family and the driver's doctor. For example, in the case where the vehicle 100 is used for a game such as a motorcycle race, the relevant party may be a team member, a team leader, or a team coach of a team to which the driver belongs.

The server apparatus 200 includes a memory 201, a controller 202, and a communication interface 203.

The memory 201 may be semiconductor memory, magnetic memory, or the like. The memory 201 stores various information, programs for operating the server apparatus 200, and the like. The memory 201 may function as working memory. The memory 201 may store information acquired from the vehicle 100. For example, the memory 201 may store the determination result of whether the movement of the body of the driver is normal.

The controller 202 includes at least one processor 202a that controls and manages the whole server apparatus 200, e.g. each functional block in the server apparatus 200. The controller 202 includes at least one processor 202a such as a CPU that executes a program defining a control procedure, to achieve its functions. Such a program is, for example, stored in the memory 201 or an external storage medium connected to the server apparatus 200. As the specific structure of the processor 202a, any of the structures listed in the description of the processor 104a may be used.

The communication interface 203 transmits and receives various information through communication with the vehicle 100. The communication interface 203 can perform information transmission and reception using a wireless network, a wire network, or a combination thereof. The communication interface 203 can perform communication using, for example, Bluetooth®, infrared, NFC, wireless LAN, wire LAN, or any other communication medium, or any combination thereof.

The determination process performed by the controller 104 will be described in detail below, together with patterns of the movement of the body of the driver.

An example in which the movement of the body of the driver is the behavior of the driver will be described first. Human behavior has a pattern specific to each individual. This pattern is also referred to as "habit". In the case where there is no physical abnormality of the driver, the behavior of the driver usually follows the pattern of the driver. However, in the case where there is a physical abnormality such as abnormal physical condition or in the case where there is a mental abnormality such as anxiety, the behavior of the driver tends to deviate from the pattern of the driver. In the case where the movement of the body of the driver is the behavior of the driver, the memory 103 stores a pattern relating to usual behavior of the driver, and the controller 104 determines whether the behavior of the driver matches the usual pattern to determine whether the behavior of the driver is a normal pattern. Herein, the term "abnormality" denotes a state different from usual, such as a state in which the driver has a disease. The term "normal pattern" denotes a pattern of usual behavior of the driver.

Prior to use of the information processing system 1, the controller 104 performs a learning (storage) process of storing at least a normal pattern of the behavior of the driver in the memory 103. The learning process may be performed by any method. As an example, the vehicle 100 can perform the following normal pattern learning process. When the driver comes within a distance in which information acquisition by the information acquisition unit 101 is possible, the controller 104 detects the position of the driver. The controller 104 detects the behavior of the driver until the driver sits on the seat 114, i.e. the change in the position of the driver. The controller 104 can determine whether the driver sits on the seat 114, based on whether the weight sensor 102 detects a load. The controller 104 may determine whether the driver sits on the seat 114, based on whether the distance between the information acquisition unit 101 and the driver acquired by the information acquisition unit 101 is equal to the distance between the information acquisition unit 101 and the driver in the case where the driver sits on the seat 114. In the case where the controller 104 determines that the driver sits on the seat 114, the controller 104 produces, on the display of the vehicle 100, a display requesting input of the driver on whether the behavior is a normal pattern. The driver provides input on whether the behavior is a normal pattern, using the input interface 105. For example, in the case where the driver thinks that his or her behavior is usual behavior, the driver can provide input indicating that the behavior is a normal pattern. For example, in the case where the driver thinks that he or she is in bad physical condition, the driver can provide input indicating that the behavior is an abnormal pattern. For example, in the case where the driver thinks that his or her behavior is different from usual behavior, the driver can provide input indicating that the behavior is an abnormal pattern. Examples of the behavior different from usual behavior include the case where the driver stumbles along the way and the case where the driver notices that he or she forgot something and turns back. The controller 104 stores the behavior which the user has input as normal, in the memory 103 as a normal pattern of the behavior. The controller 104 may store the behavior which the user has input as abnormal, in the memory 103 as an abnormal pattern of the behavior. The controller 104 may end the normal pattern learning process, in the case where pattern-related data is stored in the memory 103 to such an extent that enables determination of whether the behavior of the user is a normal pattern. For example, the controller 104 may end the normal pattern learning process in the case where the number of samples for patterns relating to the behavior of the user exceeds a predetermined number.

In the case where at least a normal pattern is stored in the memory 103, the controller 104 can perform the determination process of whether the behavior of the user is the normal pattern. The controller 104 can determine whether the behavior of the driver is the normal pattern, by comparing the behavior of the driver acquired by the information acquisition unit 101 and the pattern stored in the memory 103. For example, suppose the normal pattern of the behavior of the driver is stored in the memory 103. In the case where the controller 104 determines that the behavior of the driver acquired by the information acquisition unit 101 is within a range of the normal pattern stored in the memory 103, the controller 104 can determine that the behavior of the driver is the normal pattern. The range of the normal pattern (normal pattern range) may include not only the normal pattern stored in the memory 103 but also at least one pattern similar to the normal pattern. Whether the behavior of the driver is within the normal pattern range may be determined based on whether the behavior of the driver is within a range of a predetermined threshold with respect to the normal pattern stored in the memory 103. In the case where the controller 104 determines that the behavior of the driver acquired by the information acquisition unit 101 is not within the range of the normal pattern stored in the memory 103, the controller 104 can determine that the behavior of the driver is not a normal pattern, that is, the behavior of the driver is an abnormal pattern. The controller 104 may determine whether the behavior of the driver until he or she sits on the seat 114 is within a normal pattern.

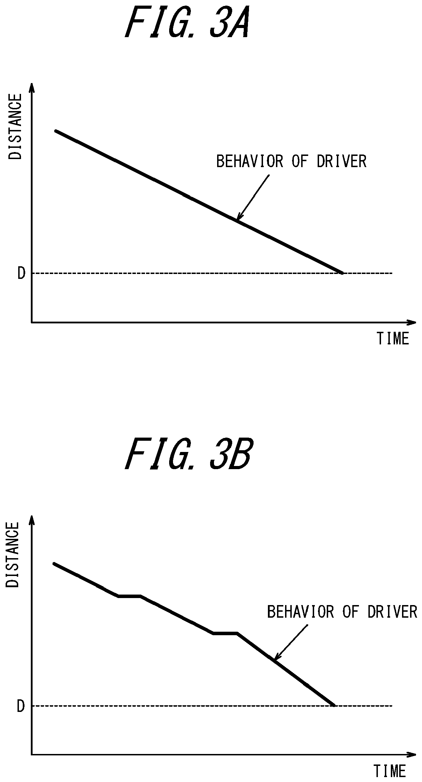

FIGS. 3A and 3B are each a diagram illustrating an example of the behavior of the driver. FIGS. 3A and 3B are each a diagram illustrating an example of the behavior of the driver that can be included in the normal pattern range. In FIGS. 3A and 3B, the horizontal axis represents time, and the vertical axis represents the distance between the driver and the information acquisition unit 101. In FIGS. 3A and 3B, distance D is the distance between the driver and the information acquisition unit 101 in a state in which the driver sits on the seat 114. FIG. 3A illustrates an example in which the driver approaches the seat 114 straight on. FIG. 3B illustrates an example in which the driver stops twice for a short time while approaching the seat 114. As an example, in the case where the patterns illustrated in FIGS. 3A and 3B are each stored as a normal pattern, if the behavior of the driver acquired by the information acquisition unit 101 is as illustrated in FIG. 3A or 3B, the controller 104 determines that the behavior of the driver is within the normal pattern range. As another example, in the case where the pattern illustrated in FIG. 3A is stored as a normal pattern and the pattern illustrated in FIG. 3B is included in the normal pattern range as a pattern similar to the pattern in FIG. 3A, if the behavior of the driver acquired by the information acquisition unit 101 is as illustrated in FIG. 3A or 3B, the controller 104 determines that the behavior of the driver is within the normal pattern range.

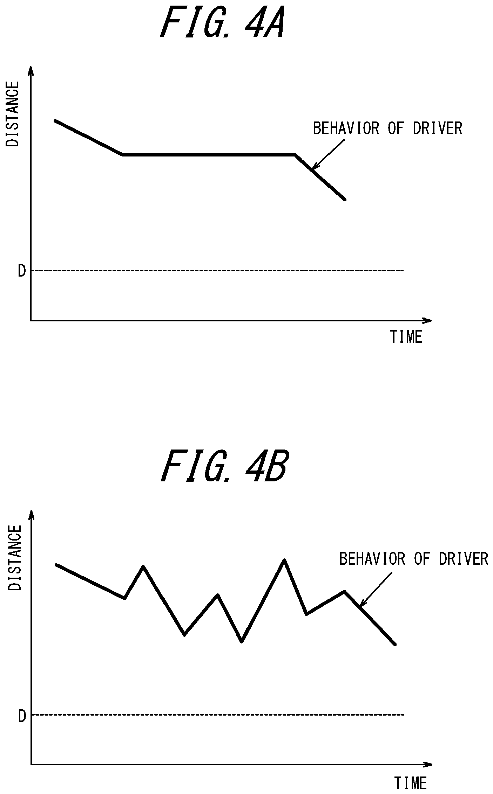

FIGS. 4A and 4B are each a diagram illustrating an example of the behavior of the driver. FIGS. 4A and 4B are each a diagram illustrating an example of the behavior of the driver that is not included in the normal pattern range. In FIGS. 4A and 4B, the horizontal axis represents time, and the vertical axis represents the distance between the driver and the information acquisition unit 101. In FIGS. 4A and 4B, distance D is the distance between the driver and the information acquisition unit 101 in a state in which the driver sits on the seat 114. In FIG. 4A, the driver stops for a long time while approaching the seat 114. In FIG. 4B, the driver repeatedly approaches or moves away from the seat 114. As an example, in the case where the pattern illustrated in FIG. 3A is stored as a normal pattern and the patterns illustrated in FIGS. 4A and 4B are not included in the normal pattern range as similar to the pattern in FIG. 3A, if the behavior of the driver acquired by the information acquisition unit 101 is as illustrated in FIG. 4A or 4B, the controller 104 determines that the behavior of the driver is not within the normal pattern range.

The memory 103 may store, as a normal pattern, a range of time usually taken from when the driver comes within the distance in which information acquisition by the information acquisition unit 101 is possible to when the driver sits on the seat 114. In this case, the controller 104 may measure the time from when the driver comes within the distance in which information acquisition by the information acquisition unit 101 is possible to when the driver sits on the seat 114, and, in the case where the time is within the range of time stored in the memory 103, determine that the behavior of the driver is the normal pattern.

The controller 104 may notify the determination result about the behavior of the driver from the notification interface 106. The controller 104 may notify from the notification interface 106 that the behavior of the driver is an abnormal pattern, only in the case where the controller 104 determines that the behavior of the driver is an abnormal pattern. The notification allows the driver to know whether his or her behavior is a normal pattern or an abnormal pattern. For example, in the case where the driver has a physical abnormality which he or she is not aware of, the driver can notice the possibility of having a physical abnormality as a result of being notified from the notification interface 106 that the behavior is an abnormal pattern.

The controller 104 can transmit the determination result about the behavior of the driver to the server apparatus 200 via the communication interface 107. The controller 104 may transmit the determination result to the server apparatus 200, only in the case where the controller 104 determines that the behavior of the driver is an abnormal pattern. The server apparatus 200 stores the determination result acquired from the vehicle 100, in the memory 201. The server apparatus 200 may store the acquired determination result in the memory 201, in association with identification information such as an ID for uniquely identifying the driver. The server apparatus 200 can store determination results relating to a plurality of drivers. The server apparatus 200 may transmit the determination result to a terminal apparatus possessed by a party relevant to the driver.

An example in which the movement of the body of the driver is the movement of the body surface caused by biological processes of the driver will be described next. For example, biological processes such as heartbeat and breathing are usually within a certain range, for each driver. In the case where there is an abnormality such as a disease, however, the biological processes may deviate from the usual range. In the case where the movement of the body of the driver is the movement of the body surface caused by biological processes of the driver, the memory 103 stores a pattern of the movement of the body surface caused by usual biological processes of the driver, and the controller 104 determines whether the movement of the body surface of the driver matches the usual pattern to determine whether the movement of the body surface of the driver is a normal pattern.

Prior to use of the information processing system 1, the controller 104 performs a learning process of storing at least a normal pattern of the movement of the body surface of the driver in the memory 103. The learning process may be performed by any method. As an example, in the case where the movement of the body of the driver is the movement of the body surface caused by biological processes of the driver, the driver may ride the vehicle 100 for a test run, to acquire a normal pattern in the test run. For example, the test run may be conducted when the driver thinks that his or her physical state is normal. In the test run, the information acquisition unit 101 acquires a pattern of the movement of the body surface of the driver. For example, the information acquisition unit 101 acquires the movement of the chest of the driver.

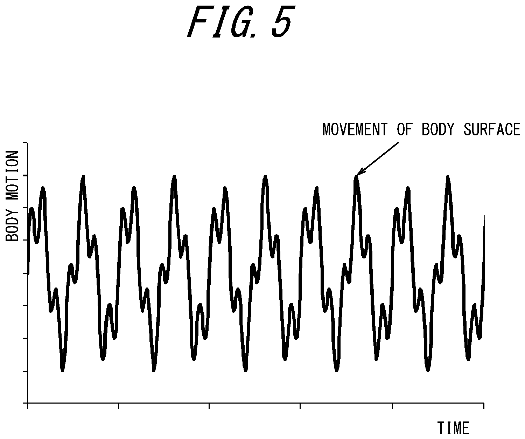

FIG. 5 is a diagram illustrating an example of a pattern of the movement of the body surface of the driver (the rise and fall or body motion of the body surface due to heartbeat, breathing, and the like) acquired by the information acquisition unit 101. FIG. 5 illustrates an example of the movement of the body surface of the driver acquired in the test run, which is stored in the memory 103 as a normal pattern. In FIG. 5, the horizontal axis represents time, and the vertical axis represents the body motion of the driver. The body surface of the driver moves due to heartbeat, breathing, and the like. The information acquisition unit 101 captures this movement as illustrated in FIG. 5. The controller 104 stores the movement of the body surface of the driver acquired in the test run, in the memory 103 as a normal pattern.

In the case where a normal pattern is stored in the memory 103, the controller 104 can perform the determination process of whether the behavior of the driver is the normal pattern. The controller 104 can determine whether the movement of the body surface of the driver is the normal pattern, by comparing the movement of the body surface of the driver acquired by the information acquisition unit 101 and the pattern stored in the memory 103. For example, in the case where the controller 104 determines that the movement of the body surface of the driver acquired by the information acquisition unit 101 is within a range of the normal pattern stored in the memory 103, the controller 104 can determine that the movement of the body surface of the driver is the normal pattern. The range of the normal pattern (normal pattern range) may include not only the normal pattern stored in the memory 103 but also at least one pattern similar to the normal pattern. Whether the movement of the body surface of the driver is within the normal pattern range may be determined based on whether the movement of the body surface of the driver is within a range of a predetermined threshold with respect to the normal pattern stored in the memory 103. In the case where the controller 104 determines that the movement of the body surface of the driver acquired by the information acquisition unit 101 is not within the range of the normal pattern stored in the memory 103, the controller 104 can determine that the movement of the body surface of the driver is not a normal pattern, that is, the movement of the body surface of the driver is an abnormal pattern.

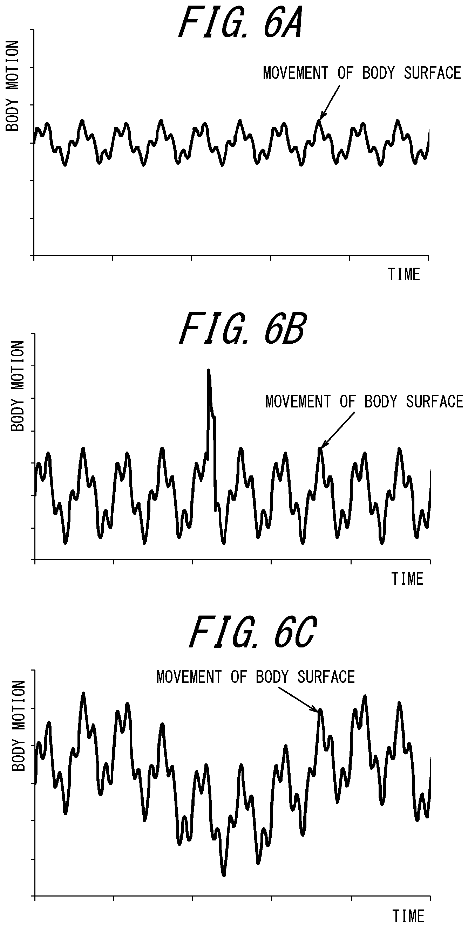

FIGS. 6A, 6B, and 6C are each a diagram illustrating an example of the movement of the body surface of the driver. FIGS. 6A, 6B, and 6C are each a diagram illustrating an example of the movement of the body surface of the driver that is not included in the normal pattern range. In FIGS. 6A, 6B, and 6C, the horizontal axis represents time, and the vertical axis represents body motion.

In FIG. 6A, the movement of the body surface of the driver is smaller than that in FIG. 5. That is, in the case where the movement of the body surface of the driver is as illustrated in FIG. 6A, the movement of the body surface of the driver is smaller than that in FIG. 5. This indicates that the strength of heartbeat of the driver weakens or the depth of breathing of the driver becomes shallower. Hence, in the case where the pattern illustrated in FIG. 5 is stored as a normal pattern, when the information acquisition unit 101 detects the movement of the body surface illustrated in FIG. 6A, the controller 104 can determine that the movement of the body surface of the driver is an abnormal pattern.

In FIG. 6B, there is a projecting part in the movement of the body surface of the driver. This indicates pumping different from usual in the heartbeat of the driver. Hence, in the case where the pattern illustrated in FIG. 5 is stored as a normal pattern, when the information acquisition unit 101 detects the movement of the body surface illustrated in FIG. 6B, the controller 104 can determine that the movement of the body surface of the driver is an abnormal pattern.

In FIG. 6C, the body surface of the driver approaches or moves away from the information acquisition unit 101. This indicates that the upper body of the driver moves back and forth. Hence, in the case where the pattern illustrated in FIG. 5 is stored as a normal pattern, when the information acquisition unit 101 detects the movement of the body surface illustrated in FIG. 6C, the controller 104 can determine that the movement of the body surface of the driver is an abnormal pattern.

The controller 104 may notify the determination result about the movement of the body surface of the driver from the notification interface 106, as described with regard to the determination process for the behavior of the driver. The controller 104 may transmit the determination result about the movement of the body surface of the driver to the server apparatus 200 via the communication interface 107.

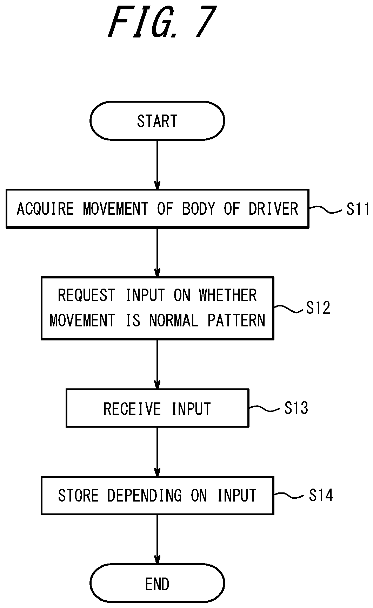

FIG. 7 is a flowchart illustrating an example of a process performed by the controller 104 in the vehicle 100, specifically, a flowchart illustrating an example of the learning (storage) process. FIG. 7 is a flowchart illustrating an example of the learning process performed in the case where the movement of the body of the driver is the behavior of the driver

First, the controller 104 acquires the movement of the body of the driver by the information acquisition unit 101 (step S11). Specifically, the controller 104 acquires the behavior of the driver.

The controller 104 requests input of the driver on whether the behavior acquired in step S11 is a normal pattern (step S12). Specifically, for example, the controller 104 produces, on the display of the vehicle 100, a display requesting input of the driver on whether the behavior acquired in step S11 is a normal pattern, to request input of the driver.

When the driver provides input from, for example, the input interface 105 in response to the request in step S12, the controller 104 receives the input by the input interface 105 (step S13).

The controller 104 stores (learns) a pattern in the memory 103, depending on the received input. For example, in the case where the input of the driver indicates that the behavior is a normal pattern, the controller 104 stores (learns) the movement of the body acquired in step S11 in the memory 103 as a normal pattern. For example, in the case where the input of the driver indicates that the behavior is an abnormal pattern, the controller 104 stores (learns) the movement of the body acquired in step S11 in the memory 103 as an abnormal pattern. The controller 104 may store (learn) the movement of the body acquired in step S11 in the memory 103, only in the case where the input of the driver indicates that the behavior is a normal pattern.

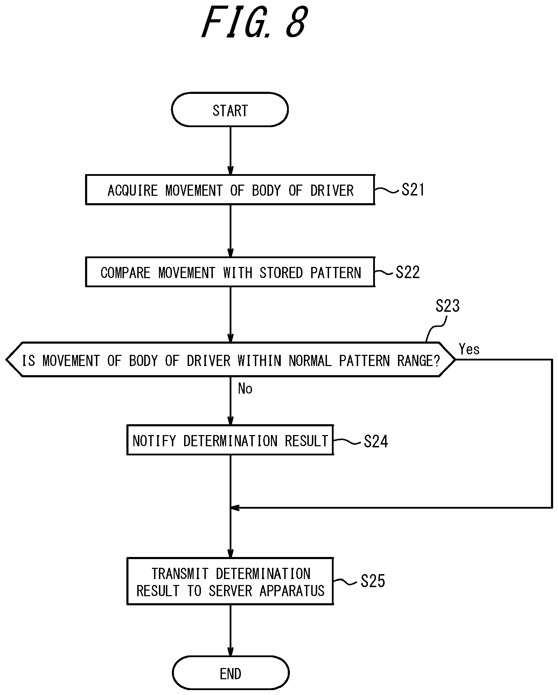

FIG. 8 is a flowchart illustrating an example of a process performed by the controller 104 in the vehicle 100, specifically, a flowchart illustrating an example of the determination process. The process of the flowchart in FIG. 8 can be performed, for example, in the case where the movement of the body of the driver is the behavior of the driver or the movement of the body surface of the driver.

First, the controller 104 acquires the movement of the body of the driver by the information acquisition unit 101 (step S21).

The controller 104 compares the movement of the body of the driver acquired in step S21 with a pattern stored in the memory 103 (step S22).

The controller 104 determines whether the movement of the body of the driver is within the normal pattern range, based on the comparison in step S22 (step S23).

In the case where the controller 104 determines that the movement of the body of the driver is within the normal pattern range (step S23: Yes), the controller 104 transmits the determination result to the server apparatus 200 (step S25).

In the case where the controller 104 determines that the movement of the body of the driver is not within the normal pattern range (step S23: No), that is, in the case where the controller 104 determines that the movement of the body of the driver is an abnormal pattern, the controller 104 notifies the determination result from the notification interface 106 (step S24). For example, the controller 104 notifies that an abnormal pattern is detected, as the determination result.

The controller 104 then transmits the determination result to the server apparatus 200 (step S25).

In the flowchart in FIG. 8, the controller 104 notifies the determination result only in the case where the controller 104 determines that the movement of the body of the driver is not within the normal pattern range (step S23: No). The controller 104 may notify the determination result in the case where the controller 104 determines that the movement of the body of the driver is within the normal pattern range (step S23: Yes).

Thus, with the information processing system 1 according to this embodiment, whether the movement of the body of the driver is a normal pattern is determined with reference to the pattern stored in the memory 103. The controller 104 can determine the state of the driver, based on whether the movement of the body of the driver is a normal pattern. The information processing system 1 therefore has improved usefulness.

Moreover, the movement of the body of the driver is acquired by the information acquisition unit 101 mounted in the vehicle 100. Thus, with the information processing system 1, the movement of the body of the driver can be acquired without the driver wearing a sensor or the like on his or her body. With the information processing system 1, the state of the driver can be detected without causing trouble and burden of wearing a sensor or the like on the driver. In addition, the movement of the body can be acquired without the driver being aware that data is being acquired. For example, in the case where the driver wears a sensor or the like on his or her body, a displacement of the sensor or the like may cause a decrease in data detection accuracy. The information processing system 1 does not have such a problem.

In order to disclose the present disclosure fully and clearly, some embodiments have been described above. However, the appended claims should not be limited to the above-described embodiments, and should be configured to embody all modifications and alternatives that can be created by those skilled in the art within the scope of the basic matters described herein. Elements in some embodiments can be combined freely.

For example, the normal pattern learning (storage) process is not limited to that described in the foregoing embodiment. The normal pattern learning (storage) process may be performed using, for example, deep learning or the like.

For example, whether the movement of the body of the driver acquired by the information acquisition unit 101 is within the range of the normal pattern stored in the memory 103 may be determined using a plurality of levels of thresholds. Whether the movement of the body of the driver acquired by the information acquisition unit 101 is within the range of the normal pattern stored in the memory 103 may be determined using, for example, two levels of thresholds.

Specifically, for example, the controller 104 may determine whether the behavior of the driver is within the range of the normal pattern stored in the memory 103, using two levels of thresholds: a first range and a second range. The first range may be narrower than the second range. In detail, the first range may be closer to the normal pattern than the second range. In the case where the behavior of the driver is within the first range, the controller 104 may determine that the behavior of the driver is a normal pattern. In the case where the behavior of the driver is not within the second range, the controller 104 may determine that the behavior of the driver is an abnormal pattern. In the case where the behavior of the driver is between the first range and the second range, the controller 104 may combine other elements to determine whether the behavior of the driver is a normal pattern.

Likewise, for example, the controller 104 may determine whether the movement of the body surface of the driver is within the range of the normal pattern stored in the memory 103, using two levels of thresholds: a third range and a fourth range. The third range may be narrower than the fourth range. In detail, the third range may be closer to the normal pattern than the fourth range. In the case where the movement of the body surface of the driver is within the third range, the controller 104 may determine that the movement of the body surface of the driver is a normal pattern. In the case where the movement of the body surface of the driver is not within the fourth range, the controller 104 may determine that the movement of the body surface of the driver is an abnormal pattern. In the case where the movement of the body surface of the driver is between the third range and the fourth range, the controller 104 may combine other elements to determine whether the behavior of the driver is a normal pattern.

For example, in the case where the behavior of the driver is between the first range and the second range and the movement of the body surface of the driver is between the third range and the fourth range, the controller 104 may determine that the movement of the body of the driver is a normal pattern. For example, in the case where the behavior of the driver is not between the first range and the second range or in the case where the movement of the body surface of the driver is not between the third range and the fourth range, the controller 104 may determine that the movement of the body of the driver is not a normal pattern.

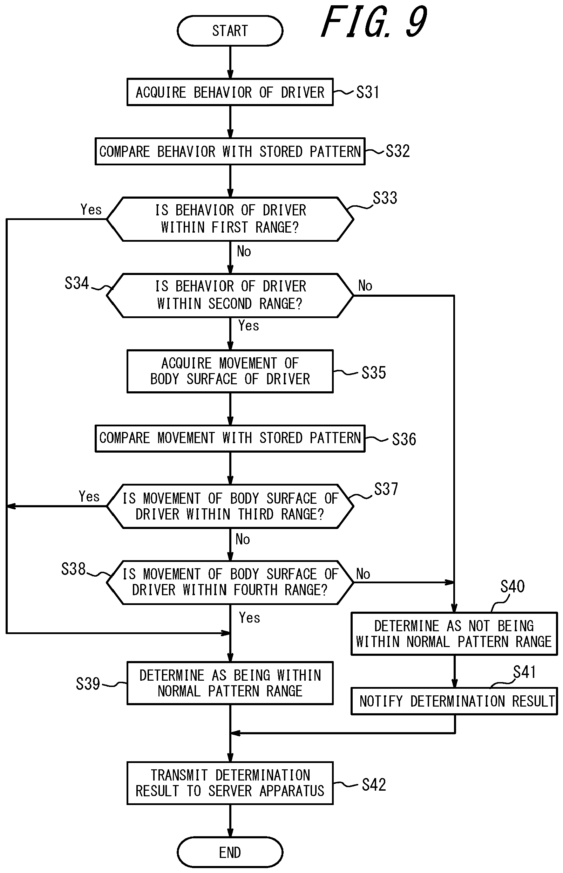

FIG. 9 is a flowchart illustrating an example of a process performed by the controller 104 in the vehicle 100, specifically, a flowchart illustrating an example of the determination process performed using the foregoing two levels of thresholds.

First, the controller 104 acquires the behavior of the driver by the information acquisition unit 101 (step S31).

The controller 104 compares the behavior of the driver acquired in step S31 with a pattern stored in the memory 103 (step S32).

The controller 104 determines whether the behavior of the driver is within the first range, based on the comparison in step S32 (step S33).

In the case where the controller 104 determines that the behavior of the driver is within the first range (step S33: Yes), the controller 104 determines that the behavior of the driver is within the normal pattern range (step S39). In this case, the controller 104 transmits the determination result to the server apparatus 200 (step S42).

In the case where the controller 104 determines that the behavior of the driver is not within the first range (step S33: No), the controller 104 determines whether the behavior of the driver is within the second range (step S34).

In the case where the controller 104 determines that the behavior of the driver is not within the second range (step S34: No), the controller 104 determines that the behavior of the driver is not within the normal pattern range (step S40). In this case, the controller 104 notifies, from the notification interface 106, that an abnormal pattern is detected, as the determination result (step S41). The controller 104 then transmits the determination result to the server apparatus 200 (step S42).

In the case where the controller 104 determines that the behavior of the driver is within the second range (step S34: Yes), the controller 104 acquires the movement of the body surface of the driver by the information acquisition unit 101 (step S35).

The controller 104 compares the movement of the body surface of the driver acquired in step S35 with a pattern stored in the memory 103 (step S36).

The controller 104 determines whether the movement of the body surface of the driver is within the third range, based on the comparison in step S36 (step S37).

In the case where the controller 104 determines that the movement of the body surface of the driver is within the third range (step S37: Yes), the controller 104 determines that the movement of the body surface of the driver is within the normal pattern range (step S39). In this case, the controller 104 transmits the determination result to the server apparatus 200 (step S42).

In the case where the controller 104 determines that the movement of the body surface of the driver is not within the third range (step S37: No), the controller 104 determines whether the movement of the body surface of the driver is within the fourth range (step S38).

In the case where the controller 104 determines that the movement of the body surface of the driver is not within the fourth range (step S38: No), the controller 104 determines that the movement of the body surface of the driver is not within the normal pattern range (step S40). In this case, the controller 104 notifies, from the notification interface 106, that an abnormal pattern is detected, as the determination result (step S41). The controller 104 then transmits the determination result to the server apparatus 200 (step S42).

In the case where the controller 104 determines that the movement of the body surface of the driver is within the fourth range (step S38: Yes), the controller 104 determines that the movement of the body of the driver is within the normal pattern range (step S39). In this case, the controller 104 transmits the determination result to the server apparatus 200 (step S42).

Thus, the controller 104 can perform the determination process using two levels of thresholds. In the case where whether the movement of the body of the driver is a normal pattern cannot be clearly determined using one item, the controller 104 can perform comprehensive determination using a plurality of items.

For example, since the controller 104 can detect the heart rate or the breathing rate by the microwave radar as described above, the controller 104 can directly determine the physical condition of the driver from the detected heart rate or breathing rate. The controller 104 can perform comprehensive determination using a plurality of items including the heart rate or the breathing rate and the pattern of the movement of the body of the driver.

In the foregoing embodiment, the controller 104 may adjust the transmission power of the microwave radar which functions as the information acquisition unit 101, to power with which the driver is detectable.

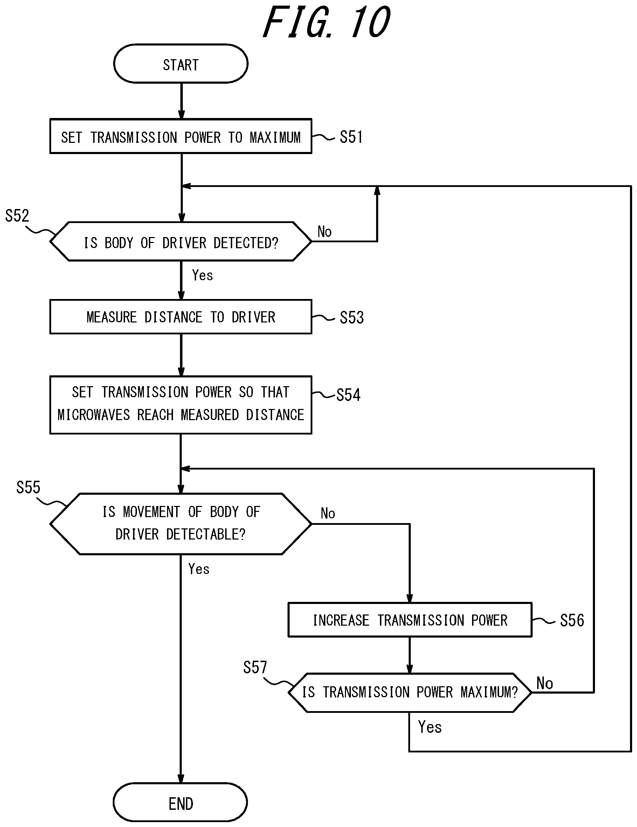

FIG. 10 is a flowchart illustrating an example of a process performed by the controller 104 in the vehicle 100, specifically, a flowchart illustrating an example of a process performed in the case of adjusting the transmission power of the microwave radar which functions as the information acquisition unit 101. Suppose the driver has not been detected by the microwave radar at the start of the flowchart in FIG. 10.

The controller 104 sets the transmission power of the microwave radar to maximum (step S51). The microwave radar can thus detect the driver as far as possible.

The controller 104 determines whether the body of the driver is detected by the microwave radar (step S52).

In the case where the controller 104 determines that the body of the driver is not detected (step S52: No), the maximum transmission power is maintained until the controller 104 determines that the body of the driver is detected.

In the case where the controller 104 determines that the body of the driver is detected (step S52: Yes), the controller 104 measures the distance to the object (driver) by the information acquisition unit 101 (step S53).

The controller 104 sets the transmission power of the microwave radar to such transmission power with which microwaves reach the distance measured in step S53 (step S54).

The controller 104 determines whether the movement of the body of the driver is detectable with the transmission power set in step S54 (step S55).

In the case where the controller 104 determines that the movement of the body of the driver is detectable (step S55: Yes), the process of the flowchart ends, and microwave transmission is continued with the transmission power set in step S54. In this case, the controller 104 may repeat the process of the flowchart from step S53. Herein, the expression "the movement of the body of the driver is detectable" denotes that data sufficient for comparison with the pattern stored in the memory 103 can be acquired.

In the case where the controller 104 determines that the movement of the body of the driver is not detectable (step S55: No), the controller 104 increases the transmission power of the microwave radar (step S56). In this case, the controller 104 may, for example, increase the transmission power of the microwave radar by a predetermined level. The predetermined level may be a predetermined power width, such as 1 dB.

The controller 104 determines whether the transmission power of the microwave radar increased in step S56 is the maximum power transmittable by the microwave radar (step S57).

In the case where the controller 104 determines that the transmission power of the microwave radar is the maximum power transmittable by the microwave radar (step S57: Yes), the controller 104 goes to step S52.

In the case where the controller 104 determines that the transmission power of the microwave radar is not the maximum power transmittable by the microwave radar (step S57: No), the controller 104 goes to step S55.

Thus, the controller 104 can adjust the transmission power of the microwave radar. As a result of the controller 104 adjusting the transmission power of the microwave radar in this way, the controller 104 can save power consumption while detecting the body of the driver.

The processes performed by the controller 104 in the vehicle 100 in the foregoing embodiment may not necessarily be performed by the controller 104 in the vehicle 100. For example, the learning process and the determination process may be performed by the controller 202 in the server apparatus 200. In this case, the movement of the body of the driver acquired by the information acquisition unit 101 is transmitted from the vehicle 100 to the server apparatus 200. In the server apparatus 200, the controller 202 can perform the learning process and the determination process. In this case, a normal pattern may be stored in the memory 201 in the server apparatus 200.

### REFERENCE SIGNS LIST

- 1: information processing system
- 100: vehicle
- 101: information acquisition unit
- 102: weight sensor
- 103, 201: memory
- 104, 202: controller
- 104a, 202a: processor
- 105: input interface
- 106: notification interface
- 107, 203: communication interface
- 110: vehicle body
- 111: front wheel
- 112: rear wheel
- 113: handlebar
- 114: seat
- 200: server apparatus

## Claims

1. A vehicle comprising:
an information acquisition unit configured to acquire a movement of a body of a driver; and
a controller configured to determine whether the movement of the body of the driver acquired by the information acquisition unit is a normal pattern of the driver.

2. The vehicle according to claim 1, further comprising
a memory configured to store a pattern of the movement of the body of the driver,
wherein the controller is configured to determine that the movement of the body of the driver acquired by the information acquisition unit is the normal pattern of the driver, in a case where the movement of the body of the driver acquired by the information acquisition unit is within a range of the pattern of the movement of the body of the driver stored in the memory.

3. The vehicle according to claim 1, wherein the movement of the body of the driver includes a behavior of the driver.

4. The vehicle according to claim 3, further comprising:
a seat on which the driver is to sit during driving; and
a weight sensor configured to detect a load on the seat,
wherein the controller is configured to determine whether the behavior of the driver until the controller determines that the driver sits on the seat based on an output of the weight sensor is the normal pattern of the driver.

5. The vehicle according to claim 1, wherein the movement of the body of the driver includes a movement caused by a biological process of the driver.

6. The vehicle according to claim 5, wherein the biological process includes at least one selected from heartbeat, breathing, and body motion.

7. The vehicle according to claim 1, further comprising
a notification interface configured to notify information,
wherein the controller is configured to notify a determination result about whether the movement of the body of the driver is the normal pattern of the driver, from the notification interface.

8. The vehicle according to claim 1, wherein the information acquisition unit is configured to emit electromagnetic waves.

9. A determination method performed by a controller, the determination method comprising:
acquiring a movement of a body of a driver by an information acquisition unit; and
determining whether the movement of the body of the driver acquired by the information acquisition unit is a normal pattern of the driver.

10. A determination program configured to cause a computer to:
acquire a movement of a body of a driver; and
determine whether the movement of the body of the driver acquired is a normal pattern of the driver.
